Europäisches Patentamt

European Patent Office

Office européen des brevets

(11) Publication number: **0 250 794 B1**

# EUROPEAN PATENT SPECIFICATION

(45) Date of publication of patent specification: 24.07.91  (51) Int. Cl.⁵: **C07C 319/28, C07C 321/12**

(21) Application number: 87106810.2

(22) Date of filing: 11.05.87

(54) Process and apparatus for desulfurizing organic polysulfides.

(30) Priority: 25.06.86 US 878163

(43) Date of publication of application:
07.01.88 Bulletin 88/01

(45) Publication of the grant of the patent:
24.07.91 Bulletin 91/30

(84) Designated Contracting States:
AT BE CH DE ES FR GB GR IT LI LU NL SE

(56) References cited:
FR-A- 2 579 203
US-A- 4 230 184

HOUBEN-WEYL: "Methoden der organischen
Chemie", 1955, edition 4, vol. IX, "Schwefel-,
Selen-, TellurVerbindungen", pages 87-92,
editor Eugen Müller, Georg Thieme Verlag,
Stuttgart, DE; "Herstellung und Umwandlung
von Polysulfiden"

(73) Proprietor: ATOCHEM NORTH AMERICA, INC.
(a Pennsylvania corp.)
Three Parkway
Philadelphia Pennsylvania 19102(US)

(72) Inventor: Hsing-Gan Yen, Jeffrey
RD1, Box 218C
Swedesboro, NJ 08085(US)
Inventor: Tuszynski, William Joseph
3 Evelyn Lane
Quakertown, PA 18951(US)
Inventor: Carroll, Glen Thomas
2437 Chestnut Avenue
Jeffersonville, PA 19401(US)
Inventor: Srinivas, Vijay Raju
71 Jennifer Drive
Chester Springs, PA 19425(US)

(74) Representative: Kraus, Walter, Dr. et al
Patentanwälte Kraus, Weisert & Partner
Thomas-Wimmer-Ring 15
W-8000 München 22(DE)

## Description

Background of the Invention

In deep sour gas wells, a solvent may be pumped down the annulus between the well casing and the production tubing in order to prevent blockage by sulfur deposition in the production string. The solvent flows back up through the production tubing along with the produced gases, is separated from the gas, and is recycled back to the well. As the solvent circulates, it absorbs a small amount of elemental sulfur which is produced by the wells. Since the solvent is recirculated, there is a continuous increase in its sulfur concentration. Dialkyl disulfides, alkyl sulfides, polysulfides, benzene, toluene, spindle oil, and the like have been used as solvents for controlling sulfur deposition. In order for this process to be economical, it is desirable to remove the sulfur from the solvent so that the solvent can be recycled downhole.

Many processes in the prior art are known for the extraction of dissolved sulfur from solvents. U.S. Patents 3,474,028, 3,489,677, 3,617,529, 3,748,827, 4,018,572, and 4,230,184 disclose the use of alkali metal and ammonium hydrosulfides and sulfides to remove dissolved sulfur from mineral oils. The publication of Dowling, Lesage, and Hyne ("Regeneration of Loaded Dimethyl Disulfide Based Sulfur Solvents", Alberta Sulfur Research Limited Quarterly Bulletin, Vol. XXI, No. 3 & 4, pp.30-52, October 1984 - March 1985) discloses the regeneration of dimethyl disulfide by stripping sulfur from dimethyl polysulfide in a batch operation with alkali metal and ammonium hydrosulfides and sulfides, preferably sodium sulfide. None of the above prior art references discloses the instant invention of a continuous multistage countercurrent flow reaction system.

Summary of the Invention

The present invention is directed to a process of removing sulfur from a stream of an organic polysulfide of high sulfur rank (such as dimethyl polysulfide) comprising

(a) continuously contacting said stream of organic polysulfide with a countercurrent stream of an immiscible aqueous stripping solution of at least one metallic sulfide or hydrosulfide salt (such as sodium sulfide) said continuous contacting occurring by mixing said streams in at least two, successive, multistage, direct contact-reaction zones to form at each such successive stage an aqueous phase of increased sulfur content and an organic phase containing a polysulfide of lower sulfur rank,

(b) separating said aqueous and organic streams between each direct contact-reaction zone, and thereafter directing each stream to a different zone until all zones of the system are traversed, said aqueous stream always being directed to that zone to which a polysulfide of sulfur rank higher than that in the zone already traversed is present,

(c) recovering the polysulfide of low sulfur rank after traversal of the last zone by said polysulfide, and

(d) optionally discarding said aqueous stream or recovering sulfur by precipitation from said aqueous stream after traversal of the last zone.

Furthermore, the present invention provides a multistage continuous countercurrent flow extractor for removing sulfur from an organic polysulfide of high sulfur rank comprising

a vertical column having a heavier liquid inlet at a first end and lighter liquid inlet at a second end, said first end having an outlet for said lighter liquid after traversing upwardly the length of said column and said second end having an outlet for said heavier liquid after traversing downwardly the length of said column, said liquids being immiscible,

said column containing a plurality of successive similar stages disposed longitudinally therein, each of said stages including components spaced from each other and from adjacent stage, each of said stages sequentially comprising,

a packing section,

first redistributor means,

agitating means, and

a second redistributor means,

distributor means interiorly adjacent said first and second ends for uniformly dispersing inwardly said heavier and lighter liquids respectively, and

a final packing section adjacent said distributor means devoid of a packing section adjacent thereto.

DESCRIPTION OF THE DRAWINGS

FIG 1 is a flow sheet of a process for removing sulfur from a sour gas well.

FIG 2 illustrates a multi-stage countercurrent flow vertical column useful in the process of the present invention.

FIG 3 is a flow sheet of a process for removing sulfur from a dimethyl polysulfide using a series of reactor tanks and separators in the sulfur removal process.

Detailed Description of the Invention

Although the process is illustrated herein by dimethyl polysulfide as the sulfur bearing organic component requiring desulfurization and aqueous sodium sulfide as the stripping solution, the invention broadly is a process for the removal of sulfur from an organic polysulfide by contacting it with an aqueous solution of one or more sulfide salts and/or hydrosulfide salts of the formula $Y_2S$ or $ZSH$ wherein $Y$ is selected from Group IA of the Periodic Table and a member of the group $NR_1R_2R_3R_4$ where $R_1$, $R_2$, $R_3$ and $R_4$ are independently selected from H, and alkyl of 1-20 carbons (such as methyl, butyl, cyclohexyl, and cetyl), aryl of 6-14 carbons (such as phenyl, naphthyl, and anthracenyl), and alkylaryl of 7-34 carbons (such as tolyl, dodecylphenyl, cetylphenyl, butylnaphthyl, and butylanthracenyl). $Z$ is selected from $Y$ and Group IIA of the Periodic Table. The reaction is carried out in a multi-stage, direct contact, countercurrent, continuous flow reactor system such that said aqueous sulfide salt and/or hydrosulfide salt chemically reacts with said organic polysulfide to give an aqueous polysulfide solution and an organic polysulfide of lower sulfur rank, i.e., a polysulfide wherein fewer sulfur atoms are present in each polysulfide molecule. The chemical reaction is depicted by the following equation (1):

$$R'SS_pSR' + nY_2S \rightarrow R'SS_{(p-q)}SR' + nYSS_{q/n}Y \qquad (1)$$

where $p>0$ and $q \leq p$.

Temperature and pressure do not materially affect the performance of the process while operation at ambient conditions is preferred. Key parameters which must be considered are the choice and concentration of the aqueous stripping solution, period of contact, and the molar ratio of the sulfide salt and/or hydrosulfide salt to recoverable sulfur in the organic polysulfide. The recoverable sulfur is the sulfur above rank two that is chemically incorporated into the organic polysulfide. These parameters are constrained by the requirement that the difference in the densities of the organic and aqueous phases in each separation zone be sufficient to allow efficient phase separation.

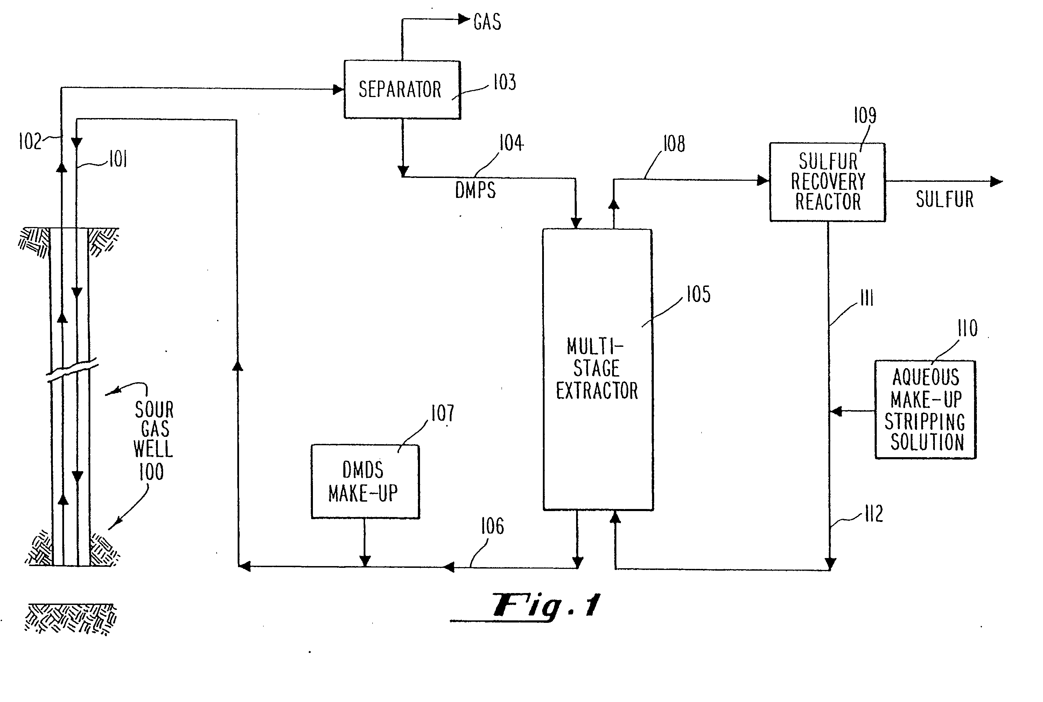

In FIGS. 1, 2 and 3, like-numbered elements of the figures are the same.

FIG 1 is a schematic flowsheet illustrating a system for sulfur removal from a sour gas well. In the processing of a sour gas well 100, sulfur often forms deposits that may plug the well and interrupt production. Such deposits may be removed by introducing a solvent for sulfur (such as dimethyl disulfide) downhole via line 101, optionally in the presence of a catalyst such as dimethyl formamide and sodium hydrosulfide, as is well known in the art. Riser pipe 102 delivers the gas and organic polysulfide (formed by reaction of the sulfur with the dimethyl disulfide) from the well bottom to separator 103 where the gas is removed from the organic polysulfide (DMPS). The gas (which is usually a mixture largely of methane, hydrogen sulfide, and carbon dioxide) is treated to separate the components and to convert the hydrogen sulfide to elemental sulfur via the well known Claus technology. The organic polysulfide is passed via line 104 to multi-stage stripping reactor represented schematically at 105 to separate elemental sulfur from the dimethyl disulfide which is returned to the well via lines 106 and 101 for reuse in well 100. Make-up dimethyl disulfide (and optionally catalyst) at 107 (DMDS) may be added to the regenerated dimethyl disulfide from reactor (or extractor) 105 to replace materials lost in processing. An aqueous stripping solution (such as sodium sulfide) is added to reactor 105 in a countercurrent flow via line 112 and, as it passes countercurrently through and reacts with the polysulfide in reactor 105, its sulfur content increases. The sulfur-laden aqueous stripping solution is discharged via line 108 to sulfur recovery system 109. Optionally, the sulfur is removed in sulfur recovery system 109 and the aqueous stripping solution may be returned via lines 111 and 112 to reactor 105. Make-up stripping solution at 110 may be added to the recycled stripping solution in lines 111 and 112 to replace material lost in processing.

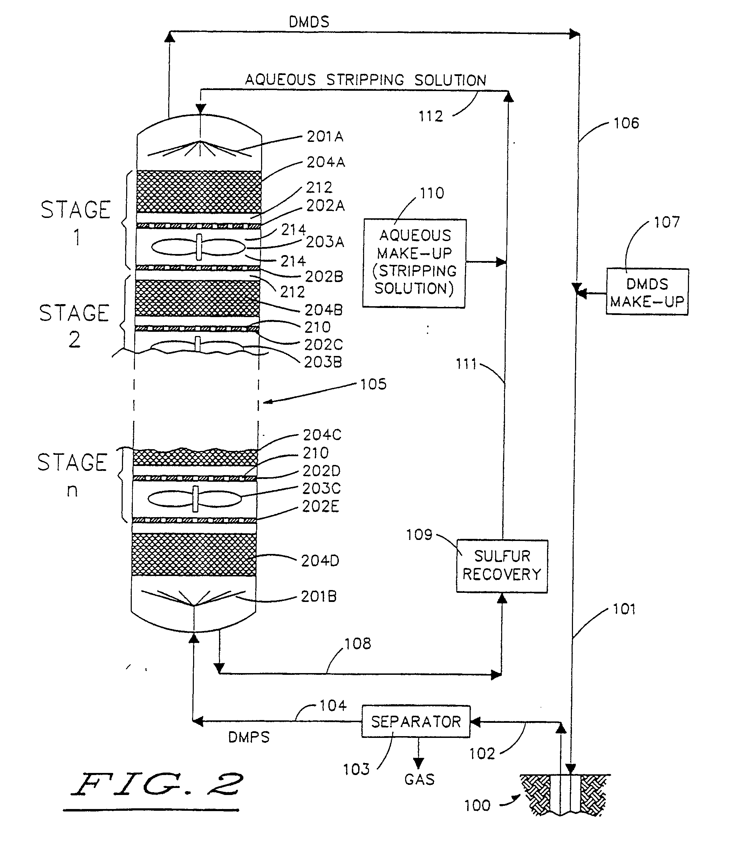

The multi-stage countercurrent flow reactor 105 may be in the form of a vertical multistage column as shown in FIG. 2 which has separate stages therein with distributors 201A and 201B, redistributor plates 202A, 202B, 202C, 202D, and 202E, agitators 203A, 203B, and 203C, and packing sections 204A, 204B, 204C, and 204D for ultimate countercurrent flow direct contact and separation. Packing section 204A, redistributor plate 202A, agitator 203A, and redistributor plate 202B comprise stage 1 of the reactor column 105. Similar components will form the other stages to the nth stage in the column as shown in Fig. 2. The circular redistribution plates 202 are provided with spaced orifices (or holes) 201 therethrough. The organic polysulfide phase is pumped into the bottom of the multistage column via line 104 while the fresh aqueous stripping solution via lines 111 and 112 flows into the top of Column 105. The aqueous stripping solution is evenly distributed cross-sectionally with the aid of a distributor 201A and similarly with the DMPS at the bottom end of the reactor column 105 by distributor 201B; the aqueous stripping solution starts to contact the sulfur-laden organic phase at the top of the column. The organic phase has a relatively lower sulfur content at the top of the column as compared to the bottom. Since the foreign sulfur content in the aqueous stripping solution is almost zero at the top of the column, the driving potential (i.e., the tendency of the chemical reaction of equation (1) to proceed from left to right) for transferring the residual recoverable sulfur from the organic phase to the aqueous phase is expected to be reasonably high. The "foreign sulfur" is the recoverable sulfur which has been transferred from the organic phase to the aqueous stripping phase.

Thereafter, the aqueous stripping solution and the organic phase are passing each other countercurrently in the packing sections 204A, 204B, 204C, and 204D. The packing sections are essential to phase separation. After the packing section 204B, the aqueous stripping solution flows through a redistributor 202C into an agitation section where both phases are stirred and mixed by agitator 203B. The agitation speed is controlled and the space 214 is reserved between the redistributor 202 and the agitators 203 and space 212 optionally between redistributor plates 202 and packing sections 204 such that the continuous upward and downward flows are maintained. Spaces 212 and 214 render the entire extraction process more efficient. The aqueous stripping solution continues to flow through the next stage including a redistributor, an agitation zone, a packing zone, and a redistributor. A number of stages can be added hereafter depending on the process needs.

Finally, the aqueous stripping solution with a high foreign sulfur loading reaches the bottom of the column 105 where the recoverable sulfur content in the organic phase is the highest throughout the column. At this point, a driving potential still exists between the aqueous stripping solution and the organic phase because of the relative concentration of sulfur in the two liquids. The sulfur-laden aqueous stripping solution is discharged from the bottom of the column via line 108 for disposal or optionally for further treatment.

The organic phase has a flow pattern similar to the aqueous stripping solution except the organic phase flows upward. If the density of the organic phase is heavier than that of the aqueous stripping solution, the above-mentioned flow pattern will be reversed.

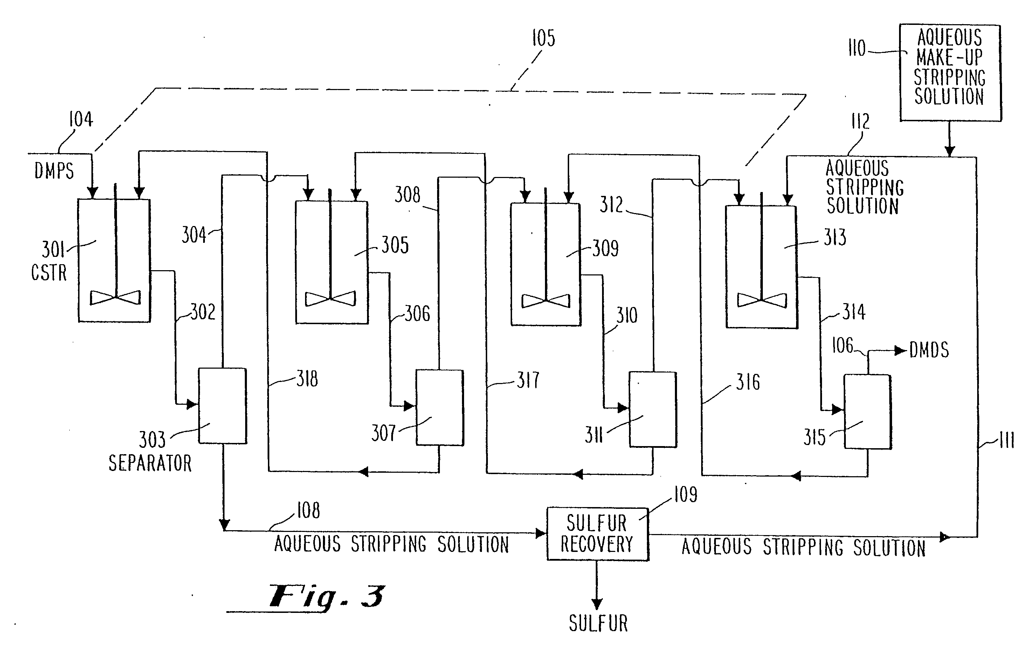

In the embodiment of FIG 3, each stage of the reactor 105 also can be in the form of a separate reactor tank 301, 305, 309, 313 with a stirrer therein and a conduit 302, 306, 310, 314 connecting each reactor tank to a separate phase separator tank 303, 307, 311, 315 where each stage is connected in series such that the organic phase from the first separator 303 will go directly into the second stage reactor tank 305 via line 304 and the organic phase from the second separator 307 should go into reactor tank 309 of the third stage via line 308 and the organic phase from the third separator 311 will go into tank 313 via line 312 and so on and so forth until the organic phase from final separator 315 is the regenerated (i.e., lower rank sulfur content polysulfide) product via line 106; and the stripping solution from each separator 307, 311, 315 is returned via lines 318, 317, 316 to the previous reactor stage 301, 305, 309 to be the stripping solution therein. In stage 313 fresh stripping solution is added thereto via lines 111 and 112 from aqueous make-up stripping solution 110 to flow countercurrently to and react with the organic polysulfide and thereafter to follow the flow pattern described above. Aqueous stripping solution containing foreign sulfur is removed from separator 303 via line 108 to be disposed of or to optionally be sent to a sulfur recovery system 109 where sulfur is removed from the aqueous stripping solution; the aqueous stripping solution may then be returned to reactor 313 via lines 111 and 112. Obviously, if the density of the organic phase is heavier than that of the aqueous stripping solution, the above-mentioned flow pattern will be reversed.

The preferred number of stages in either system is a function of the degree of regeneration and recovery required; in most cases, two stages are sufficient.

Among the sulfide salts and/or hydrosulfide salts suitable for use in the present invention, sodium sulfide in water is preferred, preferably at a concentration of between 10 weight percent and the saturation concentration of sodium sulfide at the operating temperature of the system.

The preferred reaction times (defined as the total liquid volume flow rate of the organic and aqueous phases divided into the sum of the available reaction volumes in the reactors) range from 5 to 120 minutes; generally the operation is complete in 30 minutes. At contact times shorter than 5 minutes regeneration is insufficient while contact times longer than 120 minutes do not result in significantly improved regeneration.

The molar ratio of the sulfide salt and/or hydrosulfide salt in the aqueous solution to the recoverable sulfur in the organic polysulfide (R value) may range from 0.10 to 0.70; the preferred range is 0.20 to 0.40. Using R values below 0.10 result in incomplete regeneration while using R values above 0.70 result in decreased recovery of the organic polysulfide.

The organic polysulfide does not have to originate from the downhole cleaning of a sour gas well. In the

EP 0 250 794 B1

preparation of lower organic disulfides, the disulfides are frequently separated from their co-produced polysulfides by distillation. However, it is often not feasible to purify higher organic disulfides (e.g., butyl, hexyl, nonyl, etc.) by distillation because of decomposition and the process of this invention can be employed to produce higher organic disulfides from their respective polysulfides.

Example

Employing the system of Figure 3 dimethyl polysulfide containing 25.9 weight % recoverable sulfur was reacted with a 17% aqueous solution of sodium sulfide in a continuous, countercurrent flow, direct contact two-stage system for a total of 5 minutes in the system. The molar ratio of the sodium sulfide to recoverable sulfur was 0.30. Values of 61% regeneration of the organic dimethyl disulfide and 92% recovery of the dimethyl disulfide were obtained.

For the sake of comparison, the same experiment was repeated except that a continuous single stage system was used in place of the multi-stage, countercurrent flow, direct contact system. The molar ratio of sodium sulfide to recoverable sulfur for this experiment was 0.40. Values of 61% regeneration of the organic dimethyl disulfide and 90% recovery of the dimethyl disulfide were obtained. Thus, the countercurrent, multi-stage technique of the present invention results in a savings of 25% of sodium sulfide over a single stage system.

Percent regeneration and percent recovery are defined as follows:

$$\% \ Regeneration \ = \ \frac{wt \ \% \ S_R \ (in) \ - \ wt \ \% \ S_R \ (out)}{wt \ \% \ S_R \ (in)} \ X \ 100$$

$$\% \ Recovery \ = \ \frac{weight \ disulfide \ (out)}{weight \ disulfide \ (in)} \ X \ 100 \ \%$$

where $S_R$ is the sulfur that has been chemically incorporated into the organic polysulfide.

**Claims**

1. A process of removing sulfur from a stream of an organic polysulfide of high sulfur rank comprising
   (a) continuously contacting said stream of organic polysulfide with a countercurrent stream of an immiscible aqueous stripping solution of at least one metallic sulfide or hydrosulfide salt, said continuous contacting occurring by mixing said streams in at least two, successive, multi-stage, direct contact-reaction zones to form at each such successive stage an aqueous phase of increased sulfur content and an organic phase containing a polysulfide of lower sulfur rank,
   (b) separating said aqueous and organic streams between each direct contact-reaction zone, and thereafter directing each stream to a different zone until all zones of the system are traversed, said aqueous stream always being directed to that zone to which a polysulfide of sulfur rank higher than that in the zone already traversed is present,
   (c) recovering the polysulfide of low sulfur rank after traversal of the last zone by said polysulfide, and
   (d) optionally discarding said aqueous stream or recovering sulfur by precipitation from said aqueous stream after traversal of the last zone.

2. The process of Claim 1 wherein said stripping solution has at least one salt of the formula $Y_2S$ and or $ZSH$, wherein Y is selected from Group IA of the Periodic Table or $NR_1R_2R_3R_4$ where $R_1$, $R_2$, $R_3$, and $R_4$ are independently selected from H, and alkyl of 1-20 carbons, aryl of 6-14 carbons, and alkylaryl of 7-34 carbons, and Z is selected from Y or Group IIA of the Periodic Table.

3. The process of Claim 2 wherein the number of stages of reaction zones is two.

4. The process of Claim 3 wherein the aqueous stripping solution is aqueous sodium sulfide.

5

5. The process of Claim 4 wherein the sufficient reaction time is between 5 and 120 minutes.

6. The process of Claim 5 wherein the molar ratio of the sulfide ion in the aqueous stripping solution to the recoverable sulfur in the organic polysulfide is from 0.10 to 0.70.

7. The process of Claim 1 wherein the reaction zones are continuously stirred tank reactors.

8. The process of Claim 1 wherein the reaction and separation zones are a countercurrent flow, direct contact, multi-stage vertical column wherein each stage has a mixing, packing, and redistribution zones.

9. The process of Claim 1 wherein the polysulfide is a dialkyl polysulfide.

10. The process of Claim 9 wherein the dialkyl polysulfide is dimethyl polysulfide.

11. A multistage continuous countercurrent flow extractor for removing sulfur from an organic polysulfide of high sulfur rank comprising
a vertical column (105) having a heavier liquid inlet at a first end and lighter liquid inlet at a second end, said first end having an outlet for said lighter liquid after traversing upwardly the length of said column (105) and said second end having an outlet for said heavier liquid after traversing downwardly the length of said column (105), said liquids being immiscible,
said column (105) containing a plurality of successive similar stages disposed longitudinally therein, each of said stages including components spaced from each other and from adjacent stage, each of said stages sequentially comprising,

a packing section (204A, 204B, 204C),

first redistributor means (202A, 202C, 202D),

agitating means (203A, 203B, 203C), and

a second redistributor means (202B, 202E),

distributor means (201A, 201B) interiorly adjacent said first and second ends for uniformly dispersing inwardly said heavier and lighter liquids respectively, and
a final packing section (204D) adjacent said distributor means (201B) devoid of a packing section adjacent thereto.

12. Apparatus of claim 11 wherein said lighter liquid is said organic polysulfide and said heavier liquid is an extracting or stripping solution of aqueous sodium sulfide.

13. Apparatus of claim 12 wherein said organic polysulfide and lighter liquid is dimethyl polysulfide (DMPS) and said heavier liquid is an extracting or stripping solution comprising aqueous sodium sulfide.

14. Apparatus of claim 11 wherein said heavier liquid is said organic polysulfide and said lighter liquid is an extracting or stripping solution of aqueous sodium sulfide.

15. Apparatus of claim 13 wherein said aqueous sodium sulfide extracts sulfur from said DMPS as each of said liquids travels countercurrently to the other within said column (105) to form an aqueous phase of increased sulfur content and a polysulfide of lower sulfur rank.

16. Apparatus of claim 13 wherein said liquid exiting said outlet at said second end of said column (105) is dimethyl disulfide.

**Revendications**

1. Procédé pour éliminer le soufre d'un courant d'un polysulfure organique à haute teneur en soufre, consistant
(a) à mettre en contact en continu ledit courant de polysulfure organique avec un courant, dirigé en sens opposé, d'une solution aqueuse non miscible d'épuration renfermant au moins un sulfure ou sulfhydrate métallique, ledit contact continu se produisant par mélange desdits courants dans au moins deux zones de réaction par contact direct à plusieurs étages successifs pour la formation, à chacun de ces étages successifs, d'une phase aqueuse à plus forte teneur en soufre et d'une phase organique contenant un polysulfure à plus faible teneur en soufre,

(b) à séparer ledit courant aqueux et ledit courant organique entre chaque zone de réaction par contact direct, puis à diriger chaque courant vers une zone différente jusqu'à ce que toutes les zones du dispositif soient traversées, ledit courant aqueux étant toujours dirigé vers la zone dans laquelle est présent un polysulfure à teneur en soufre plus élevée que celle de la zone déjà traversée,

(c) à séparer le polysulfure à faible teneur en soufre après traversée de la dernière zone par ledit polysulfure, et

(d) à rejeter facultativement ledit courant aqueux ou à séparer le soufre par précipitation dans ledit courant aqueux après traversée de la dernière zone.

2. Procédé suivant la revendication 1, dans lequel la solution d'épuration renferme au moins un sel de formule $Y_2S$ et/ou $ZSH$, dans laquelle Y est choisi dans le Groupe IA du Tableau Périodique ou répond à la formule $NR_1R_2R_3R_4$ dans laquelle $R_1$, $R_2$, $R_3$ et $R_4$ sont choisis, indépendamment, entre H et des groupes alkyle ayant 1 à 20 atomes de carbone, aryle ayant 6 à 14 atomes de carbone et alkylaryle ayant 7 à 34 atomes de carbone, et Z est choisi entre Y et un élément du Groupe IIA du Tableau Périodique.

3. Procédé suivant la revendication 2, dans lequel le nombre d'étages des zones de réaction est égal à deux.

4. Procédé suivant la revendication 3, dans lequel la solution aqueuse d'épuration est une solution aqueuse de sulfure de sodium.

5. Procédé suivant la revendication 4, dans lequel le temps suffisant de réaction est compris dans l'intervalle de 5 à 120 minutes.

6. Procédé suivant la revendication 5, dans lequel le rapport molaire de l'ion sulfure dans la solution aqueuse d'épuration au soufre séparable dans le polysulfure organique va de 0,10 à 0,70.

7. Procédé suivant la revendication 1, dans lequel les zones de réaction consistent en réacteurs à réservoirs sous agitation continue.

8. Procédé suivant la revendication 1, dans lequel les zones de réaction et de séparation consistent en une colonne verticale à plusieurs étages, à écoulement à contre-courant et à contact direct, dans laquelle chaque étage possède une zone de mélange, une zone de garnissage et une zone de redistribution.

9. Procédé suivant la revendication 1, dans lequel le polysulfure est un polysulfure de dialkyle.

10. Procédé suivant la revendication 9, dans lequel le polysulfure de dialkyle est le polysulfure de diméthyle.

11. Extracteur à écoulement continu à contrecourant, à plusieurs étages pour l'élimination du soufre d'un polysulfure organique à haute teneur en soufre, comprenant

une colonne verticale (105) ayant un orifice d'admission de liquide plus lourd à une première extrémité et un orifice d'admission de liquide plus léger à une seconde extrémité, ladite première extrémité ayant un orifice de sortie pour ledit liquide plus léger après traversée ascendante le long de ladite colonne (105) et ladite seconde extrémité ayant un orifice de sortie pour ledit liquide plus lourd après traversée descendante le long de ladite colonne (105), lesdits liquides étant non miscibles,

ladite colonne (105) contenant plusieurs étages similaires successifs disposés longitudinalement dans cette colonne, chacun de ces étages comprenant des composants espacés les uns des autres et de l'étage adjacent, chacun de ces étages comprenant successivement

une section de garnissage (204A, 204B, 204C),

des premiers moyens de redistribution (202A, 202C, 202D),

des moyens d'agitation (203A, 203B, 203C), et

des seconds moyens de redistribution (202B, 202E),

des moyens de distribution (201A, 201B) intérieurement adjacents auxdites première et seconde extrémités pour disperser, respectivement, de manière uniforme, intérieurement, lesdits liquides plus lourd et plus léger, et

une section finale de garnissage (204D) adjacente auxdits moyens de distribution (201B) et dépourvue de section de garnissage adjacente.

12. Appareil suivant la revendication 11, dans lequel le liquide plus léger consiste en le polysulfure organique et le liquide plus lourd est une solution d'extraction ou d'épuration consistant en une solution aqueuse de sulfure de sodium.

13. Appareil suivant la revendication 12, dans lequel le polysulfure organique et le liquide plus léger consistent en polysulfure de diméthyle (DMPS) et le liquide plus lourd est une solution d'extraction ou d'épuration consistant en une solution aqueuse de sulfure de sodium.

14. Appareil suivant la revendication 11, dans lequel le liquide plus lourd consiste en le polysulfure organique et le liquide plus léger est une solution d'extraction ou d'épuration consistant en une solution aqueuse de sulfure de sodium.

15. Appareil suivant la revendication 13, dans lequel la solution aqueuse de sulfure de sodium extrait le soufre du DMPS lorsque chacun de ces liquides subit un écoulement à contre-courant de l'autre liquide à l'intérieur de la colonne (105) pour former une phase aqueuse à teneur accrue en soufre et un polysulfure à teneur réduite en soufre.

16. Appareil suivant la revendication 13, dans lequel le liquide quittant l'orifice de sortie à la seconde extrémité de la colonne (105) est le disulfure de diméthyle.

## Patentansprüche

1. Verfahren zum Entfernen von Schwefel aus einem Strom eines organischen Polysulfids von hohem Schwefelrang, umfassend:

(a) kontinuierliches Kontaktieren des Stroms von organischem Polysulfid mit einem Gegenstrom einer unvermischbaren wäßrigen Abstreiflösung von wenigstens einem metallischen Sulfid- oder Hydrosulfidsalz, wobei das kontinuierliche Kontaktieren durch Mischen der Ströme in wenigstens zwei aufeinanderfolgenden, Mehrstufen-, Direktkontakt-Reaktionszonen stattfindet, um in jeder solcher aufeinanderfolgenden Stufe eine wäßrige Phase von zunehmendem Schwefelgehalt und eine organische Phase, die ein Polysulfid von niedrigerem Schwefelrang enthält, zu bilden,

(b) Trennen des wäßrigen und des organischen Stroms zwischen jeder Direktkontakt-Reaktionszone, und danach Leiten jedes Stroms zu einer unterschiedlichen Zone, bis alle Zonen des Systems durchquert sind, wobei der wäßrige Strom stets zu derjenigen Zone geleitet wird, bei der ein Polysulfid von einem Schwefelrang, der höher ist als derjenige in der bereits durchquerten Zone, vorhanden ist.

(c) Wiedergewinnen des Polysulfids von niedrigem Schwefelrang nach dem Durchqueren der letzten Zone durch das Polysulfid, und

(d) wahlweise Wegwerfen des wäßrigen Stroms oder Wiedergewinnen des Schwefels durch Ausscheidung aus dem wäßrigen Strom nach dem Durchqueren der letzten Zone.

2. Verfahren nach Anspruch 1, worin die Abstreiflösung wenigstens ein Salz der Formel $Y_2S$ oder $ZSH$, worin Y aus der Gruppe IA des periodischen Systems ausgewählt ist, oder $NR_1R_2R_3R_4$, worin $R_1$, $R_2$, $R_3$ und $R_4$ unabhängig ausgewählt sind von H und einen Alkyl von 1-20 Kohlenstoffatomen, Aryl von 6-14 Kohlenstoffatomen, und Alkylaryl von 7-34 Kohlenstoffatomen, und worin Z von Y oder der Gruppe IIA des periodischen Systems ausgewählt ist, hat.

3. Verfahren nach Anspruch 2, worin die Anzahl der Stufen der Reaktionszonen zwei ist.

4. Verfahren nach Anspruch 3, worin die wäßrige Abstreiflösung wäßriges Natriumsulfid ist.

5. Verfahren nach Anspruch 4, worin die genügende Reaktionszeit zwischen 5 und 120 Minuten ist.

6. Verfahren nach Anspruch 5, worin das molare Verhältnis des Sulfidions in der wäßrigen Abstreiflösung zu dem wiedergewinnbaren Schwefel in dem organischen Polysulfid von 0,10 bis 0,70 ist.

7. Verfahren nach Anspruch 1, worin die Reaktionszonen kontinuierlich gerührte Tankreaktoren sind.

8. Verfahren nach Anspruch 1, worin die Reaktions- und Trennzonen eine Gegenstrom-, Direktkontakt-, Mehrstufen-Vertikalsäule sind, worin jede Stufe eine Mischungs-, Packungs- und Wiederverteilungszone hat.

9. Verfahren nach Anspruch 1, worin das Polysulfid ein Dialkyl-Polysulfid ist.

10. Verfahren nach Anspruch 9, worin das Dialkyl-Polysulfid Dimethyl-Polysulfid ist.

11. Mehrstufiger kontinuierlicher Gegenstrom-Strömungsextraktor zum Entfernen von Schwefel aus einem organischen Polysulfid von hohem Schwefelrang, umfassend:

eine vertikale Säule (105), die einen Einlaß für schwerere Flüssigkeit an einem ersten Ende und einen Einlaß für leichtere Flüssigkeit an einem zweiten Ende hat, wobei das erste Ende einen Auslaß für die leichtere Flüssigkeit nach dem Aufwärtsdurchqueren der Länge der Säule (105) hat und das zweite Ende einen Auslaß für die schwerere Flüssigkeit nach dem Abwärtsdurchqueren der Länge der Säule (105) hat, wobei die Flüssigkeiten unvermischbar sind,

wobei die Säule (105) eine Mehrzahl von aufeinanderfolgenden ähnlichen Stufen enthält, die in Längsrichtung darin angeordnet sind, wobei jede der Stufen Komponenten aufweist, die von einander und von der benachbarten Stufe beabstandet sind, wobei jede der Stufen aufeinanderfolgend folgendes umfaßt:

einen Packungsabschnitt (204A, 204B, 204C),

eine erste Wiederverteilungseinrichtung (202A, 202C, 202D),

eine Rühreinrichtung (203A, 203B, 203C), und

eine zweite Wiederverteilungseinrichtung (202B, 202E),

eine Verteilungseinrichtung (201A, 201B) innen benachbart dem ersten und zweiten Ende zum gleichförmigen einwärtigen Verteilen der schwereren bzw. leichteren Flüssigkeit, und

einen Endpackungsabschnitt (204D) benachbart der Verteilungseinrichtung (201B), die frei von einem dazu benachbarten Packungsabschnitt ist.

12. Einrichtung nach Anspruch 11, worin die leichtere Flüssigkeit das organische Polysulfid ist und die schwerere Flüssigkeit eine Extraktions- oder Abstreiflösung von wäßrigem Natriumsulfid ist.

13. Einrichtung nach Anspruch 12, worin das organische Polysulfid und die leichtere Flüssigkeit Dimethyl-Polysulfid (DMPS) ist und die schwerere Flüssigkeit eine Extraktions- oder Abstreiflösung, welche wäßriges Natriumsulfid umfaßt, ist.

14. Einrichtung nach Anspruch 11, worin die schwerere Flüssigkeit das organische Polysulfid ist und die leichtere Flüssigkeit eine Extraktions- oder Abstreiflösung von wäßrigem Natriumsulfid ist.

15. Einrichtung nach Anspruch 13, worin das wäßrige Natriumsulfid Schwefel aus der DMPS extrahiert, wenn jede der Flüssigkeiten im Gegenstrom zu der anderen innerhalb der Säule (105) wandert, so daß eine wäßrige Phase von erhöhtem Schwefelgehalt und ein Polysulfid von niedrigerem Schwefelrang gebildet werden.

16. Einrichtung nach Anspruch 13, worin die Flüssigkeit, welche aus dem Auslaß an dem zweiten Ende der Säule (105) austritt, Dimethyl-Disulfid ist.

_Fig.1_

EP 0 250 794 B1

*FIG. 2*

*Fig. 3*